(19) Europäisches Patentamt
European Patent Office
Office européen des brevets

(11) Numéro de publication: **0 018 245**
**B1**

# (12) FASCICULE DE BREVET EUROPEEN

(45) Date de publication du fascicule du brevet: **02.06.82**

(21) Numéro de dépôt: **80400332.5**

(22) Date de dépôt: **13.03.80**

(51) Int. Cl.³: **C 07 J 21/00**, C 07 J 1/00, C 07 J 51/00, A 61 K 31/565, A 61 K 31/585

## (54) Nouveaux dérivés stéroides 7-alkylés, leur procédé de préparation, leur application comme médicament et les compositions pharmaceutiques les renfermant.

(30) Priorité: **22.03.79 FR 7907273**

(43) Date de publication de la demande:
**29.10.80 Bulletin 80/22**

(45) Mention de la délivrance du brevet:
**02.06.82 Bulletin 82/22**

(84) Etats contractants désignés:
**AT BE CH DE FR GB IT LU NL SE**

(56) Documents cités:
**JOURNAL OF ORGANIC CHRMISTRY, vol. 26, n° 9, 12 septembre 1961, pages 3077—3083 Washington DC, U.S.A. N. W. ATWATER et al.: "Steroidal Aldosterone Antagonists"**

**STEROIDS, vol. 27, n° 6, juin 1976, pages 759—771 San Francisco, U.S.A. J. F. GRUNWELL et al.: "Antiprogestational agents — The synthesis of 7-alkyl steroidal ketones with anti-implantational and anti-decidual activity"**

(73) Titulaire: **ROUSSEL-UCLAF**
**35, boulevard des Invalides**
**F-75007 Paris (FR)**

(72) Inventeur: **Nedelec, Lucien**
**45, boulevard de l'Ouest**
**F-93340 Le Raincy (FR)**
Inventeur: **Torelli, Vesperto**
**5, rue de la Convention**
**F-94700 Maisons Alfort (FR)**
Inventeur: **Fournex, Robert**
**8, rue du Commandant Rivière**
**F-75008 Paris (FR)**
Inventeur: **Tournemine, Colette**
**25, allée Rémond**
**F-93190 Livry-Gargan (FR)**

(74) Mandataire: **Tonnellier, Marie-José et al,**
**102, route de Noisy Boîte postale no 9**
**F-93230 Romainville (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 018 245 B1

Nouveaux dérivés stéroïdes 7-alkylés, leur procédé de préparation, leur application comme médicament et les compositions pharmaceutiques les renfermant

La présente invention concerne de nouveaux dérivés stéroïdes 7-alkylés, leur procédé de préparation, leur application comme médicament et les compositions pharmaceutiques les renfermant.

Dans le Journal of Organic Chemistry vol. 26, N° 9 1961 pages 3077—3083 sont décrites des spirolactones antagonistes de l'aldosterone et en particulier la lactone de l'acide 3-(3-oxo 7-$\alpha$-méthyl 17$\beta$-hydroxy 4-androsten-17$\alpha$-yl) propanoïque.

Dans Stéroïds, vol. 27, N° 6, 1976 pages 759—771 sont décrits des produits pouvant comporter un radical méthyle ou alkyle en position 7 mais, ne comportant pas de cycle $\gamma$ lactone en 17 ou de radical hydroxyle en 17$\alpha$ et carboxyéthyle en 17$\beta$.

L'invention a pour objet les composés répondant à la formule I:

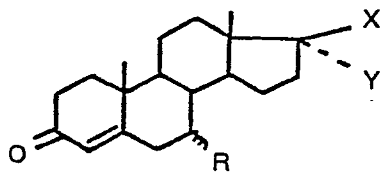

(I)

dans laquelle R, en position $\alpha$ ou en position $\beta$, représente un radical alkyle saturé ou insaturé, renfermant de 2 à 8 atomes de carbone, un radical cycloalkylalkyle renfermant de 4 à 8 atomes de carbone ou un radical arylalkyle renfermant de 7 à 12 atomes de carbone et,

— ou bien X et Y représentent le groupement:

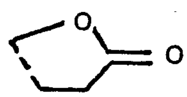

— ou bien X représente le groupement OH et Y le groupement

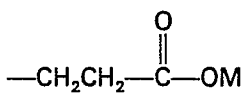

dans lequel M représente un atome d'hydrogène, un atome de métal alcalin ou un groupement $NH_4$.

Lorsque R représente un radical alkyle saturé, il s'agit de préférence du radical éthyle, propyle, isopropyle, butyle, isobutyle ou terbutyle, n-pentyle, n-hexyle, 2-méthylpentyle, 2,3-diméthylbutyle ou n-heptyle.

Lorsque R représente un radical alkyle insaturé, il s'agit, de préférence, du radical vinyle, isopropényle ou allyle, du 2-méthyl allyle, d'un radical butényle ou isobutényle.

Lorsque R représente un radical cycloalkylalkyle, il s'agit, de préférence, d'un radical cyclopropylalkyle comme, par exemple, le radical cyclopropylméthyle ou cyclopropyléthyle.

Lorsque R représente un radical arylalkyle, il s'agit de préférence du radical benzyle.

Lorsque M représente un atome de métal alcalin, il s'agit de préférence d'un atome de sodium, de potassium ou de lithium.

Parmi les composés préférés de l'invention, on peut citer les composés de formule I pour lesquels X et Y représentent le groupement

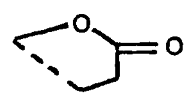

ainsi que ceux pour lesquels X représente un radical hydroxyle et Y un radical

$$-CH_2CH_2CO_2K.$$

Parmi les composés de l'invention, on peut citer tout particulièrement les composés de formule I pour lesquels le radical R est en position 7 $\alpha$, ceux pour lesquels R représente un radical éthyle, n-propyle, n-butyle ou 2-méthylpropyle ainsi que ceux pour lesquels R représente le radical vinyle ou allyle.

L'invention a naturellement tout spécialement pour objet les composés dont la préparation est donnée plus loin dans la partie expérimentale et tout particulièrement le 17$\beta$-hydroxy 3-oxo 7$\alpha$-propyl (17$\alpha$) pregn-4-ène 21-carboxylate de potassium et la Y-lactone de l'acide 17$\beta$-hydroxy 3-oxo 7$\alpha$-propyl (17$\alpha$) pregn-4-ène 21-carboxylique.

Les composés de formule I présentent de très intéressantes propriétés pharmacologiques; ils sont, en particulier, des antagonistes de l'aldostérone et augmentent la diurèse hydrosodée avec conservation du potassium organique; ils présentent, en outre, l'avantage d'être dénués d'effets

hormonaux secondaires, en particulier d'effets anti-androgène et anti-estrogène. Ils peuvent donc être utilisés pour lutter, notamment, contre l'hypertension artérielle et les insuffisances cardiaques.

Enfin, les composés de formule I se révèlent plus actifs que la lactone de l'acide 3-(3-oxo 7-$\alpha$-méthyl 17$\beta$-hydroxy 4-androsten-17$\alpha$-yl) propanoïque décrite dans le Journal of Organic Chemistry (composé 27 page 3079).

L'invention a donc pour objet les composés de formule I à titre de médicament.

Parmi les médicaments selon l'invention, on peut citer tout particulièrement les produits préférés mentionnés ci-dessus.

La posologie utile varie en fonction de l'affection à traiter et de la voie d'administration; elle peut aller, par exemple, de 10 mg à 1 g par jour chez l'adulte par voie orale. Les composés de formule I sont utilisés par voie buccale, rectale, transcutanée, ou intraveineuse. Ils peuvent être prescrits sous forme de comprimés, de comprimés enrobés, de cachets, de capsules, de granulés, d'émulsions, de sirops, de suppositoires et de préparations injectables.

L'invention a donc également pour objet les compositions pharmaceutiques renfermant comme principe actif au moins un composé de formule I. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs. Les compositions selon l'invention sont préparées selon les méthodes usuelles. Il est à noter que le principe actif utilisé peut se présenter sous forme micronisée.

L'invention a également pour objet un procédé de préparation des composés de formule I, caractérisé en ce que l'on soumet le composé de formule II:

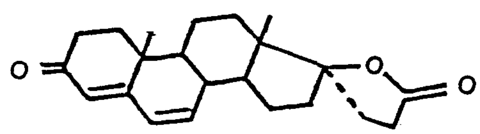

(II)

d'abord à l'action d'un composé de formule III:

RMgHal (III)

dans laquelle R conserve la même signification que précédemment et Hal représente un atome d'halogène, en présence d'un sel cuivreux, ou d'un composé de formule IV:

$(R)_2$ Cu Li (IV)

dans laquelle R conserve la même signification que précédemment, puis à l'action d'un acide, pour obtenir un composé de formule $I_A$:

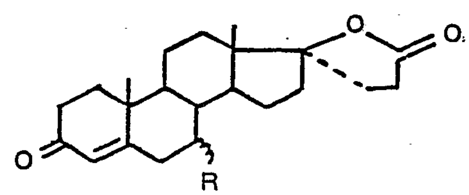

$I_A$

éventuellement sous forme d'un mélange d'isomères 7$\alpha$ et 7$\beta$ que l'on sépare, si désiré, puis, si désiré, soumet soit chacun des isomères, soit leur mélange à l'action d'un hydroxyde alcalin ou de l'ammoniaque, pour obtenir un composé de formule $I_B$:

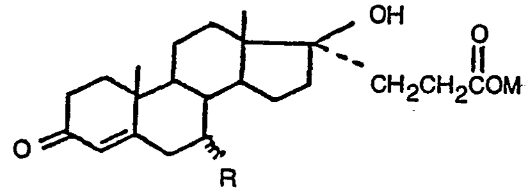

$I_B$

dans laquelle R conserve la même signification que précédemment et M représente un atome de métal alcalin ou un groupement $NH_4$, sous forme d'isomère 7$\alpha$ ou 7$\beta$, ou de leur mélange, que l'on sépare, si désiré, en chacun des isomères, puis soumet soit chacun des isomères ainsi obtenus soit leur mélange à l'action d'un agent acide pour obtenir le composé correspondant de formule $I_B$ dans laquelle M représente un atome d'hydrogène.

Dans un mode de réalisation préféré du procédé de l'invention:

— on utilise un composé de formule RMgX dans laquelle X représente un atome de chlore, de brome ou d'iode;

— on utilise comme sel cuivreux un chlorure, bromure ou iodure cuivreux;

— l'acide utilisé est un acide fort, par exemple, l'acide chlorhydrique, l'acide nitrique ou l'acide sulfurique;

— on sépare les différents isomères obtenus par chromatographie ou par cristallisation fractionnée;

— l'hydroxyde alcalin à l'action duquel on soumet le composé de formule $I_A$ est la soude ou la potasse;

— l'agent acide auquel on soumet le composé de formule $I_B$ est l'acide chlorhydrique, l'acide sulfurique, l'acide nitrique ou l'acide acétique.

Le composé de formule II utilisé comme produit de départ du procédé de l'invention est un produit connu, il peut être préparé selon le procédé décrit dans le brevet américain n° 3 194 803.

Les exemples suivant illustrent l'invention sans toutefois la limiter.

## EXEMPLE 1

γ-lactone de l'acide 7α-éthyl 17β-hydroxy 3-oxo (17α) prègn-4-ène 21-carboxylique.

On recouvre 2,86 g d'iodure cuivreux avec 5,25 cm3 de sulfure de n-butyle et agite le mélange à la température ordinaire. La dissolution du sel cuivreux, légèrement exothermique, est totale en 10 minutes environ. Au complexe ainsi formé ou ajoute une solution de 10,2 g de canrénone (ou γ-lactone de l'acide 17β-hydroxy 3-oxo (17α) prégna 4,6-diène 21-carboxylique) dans 150 cm3 de tétrahydrofuranne anhydre. La solution obtenue est refroidie à −30°C et sous bonne agitation, on introduit lentement, en 75 minutes, 54 cm3 de solution éthérée 0,9 M de bromure d'éthylmagnésium diluée avec 50 cm3 d'éther éthylique et 50 cm3 de tétrahydrofurane. On poursuit l'agitation à −30°C pendant encore 60 minutes puis acidifie le mélange réactionnel avec 120 cm3 d'acide chlorhydrique 2N. On agite 60 minutes à la température ordinaire et décante la phase organique. Elle est lavée à l'eau, sèchée et distillée à sec. Le résidu cristallisé obtenu est purifié par chromatographie sur silice en utilisant le mélange cyclohexane-acétate d'éthyle (3—2). On isole dans l'ordre:

— 6,3 g d'isomère α recherché, fondant à 167°C. Le point de fusion reste inchangé après recristallisation du produit dans du méthanol.

$[\alpha]_D = +67° \pm 1{,}5°$ (1%, chloroforme);

—3,2 g d'isomère β, fondant vers 150°C puis à 170°C. L'échantillon est obtenu par recristallisation dans du méthanol. PF = 155°C puis 172°C après resolidification.

$[\alpha]_D = +62{,}5° \pm 1{,}5°$ (1%, chloroforme).

## EXEMPLE 2

γ-lactone de l'acide 17β-hydroxy 3-oxo 7α-propyl (17α) prégn-4-ène 21-carboxylique.

Au complexe obtenu en agitant à la température ordinaire 382 mg d'iodure cuivreux et 0,7 cm3 de sulfure de n-butyle, on ajoute une solution de 5,1 g de canrénone (ou de γ-lactone de l'acide 17β-hydroxy 3-oxo (17α) pregna 4,6-diène 21-carboxylique) dans 75 cm3 de tétrahydrofuranne anhydre et 50 cm3 d'éther éthylique anhydre. On refroidit le mélange á −20°C et sous bonne agitation, introduit en 35 minutes une solution éthérée 1,2 M de bromure de propylmagnésium. Après 10 minutes d'agitation supplémentaire à −20°C, la suspension réactionnelle jaune est acidifiée avec 50 cm3 d'acide chlorhydrique 5N. On agite 30 minutes à la température ordinaire puis extrait avec de l'acétate d'éthyle. La phase organique est lavée à l'eau, avec une solution de thiosulfate de sodium 0,2N et à l'eau, puis elle est sèchée sur sulfate de soude et distillée à sec. L'extrait sec huileux renfermant les deux isomères 7α et 7β-propylés d'addition −1,6 est purifié par chromatographie sur silice en utilisant un mélange benzène-éther éthylique (1—4). On sépare dans l'ordre:

— 3,3 g d'isomère α cristallisé;

— 1,4 g d'isomère β amorphe.

L'échantillon du composé recherché (dérivé 7α-propylé) est obtenu par recristallisation dans le méthanol: $F_I$=205°C;

$[\alpha]_D = +70° \pm 1{,}5°$ (1%, chloroforme).

## EXEMPLE 3

17β-hydroxy 3-oxo 7α-propyl (17α) prègn-4-ène 21-carboxylate de potassium.

On chauffe 1,15 g du composé obtenu à l'exemple 2 au reflux d'un mélange de 5,4 cm3 de solution 0,5 3N de potasse dans l'éthanol et de 5,4 cm3 d'eau pendant 15 minutes, puis concentre à petit volume sous pression réduite jusqu'à l'obtention d'un sirop jaune épais. Par addition de 50 cm3 d'acétone, le sel de potassium cristallise. On l'essore, le lave à l'acétone et le sèche vers 50°C. Le sel de potassium brut est recristallisé par dissolution dans 1,3 cm3 d'eau et addition de 19,5 cm3 d'acétone. On obtient 1 g d'aiguilles incolores solvatées avec une molécule d'eau: F (bloc Maquenne) vers 290°C; $[\alpha]_D = +48° \pm 1{,}5°$ (1%, eau).

EXEMPLE 4

γ-lactone de l'acide 7α-butyl 17β-hydroxy 3-oxo (17α) prègn-4-ène 21-carboxylique.

On agite ensemble 191 mg d'iodure cuivreux et 0,35 cm3 de sulfure de n-butyle, puis dissout le complexe formé dans 35 cm3 de tétrahydrofuranne anhydre et ajoute 1,7 g de canrénone (ou γ-lactone de l'acide 17β-hydroxy 3-oxo (17α)-pregna 4,6-diène 21-carboxylique). On agite la solution au bain de glace (température intérieure vers + 5°C) et introduit lentement, en 30 minutes, 7,5 cm3 de solution 1,2 M de bromure de butylmagnésium dans l'éther éthylique. Après 30 minutes, on acidifie le mélange réactionnel avec un large excès d'acide chlorhydrique 5N et traite de la menière citée à l'exemple 2. Par chromatographie sur silice, en utilisant un mélange cyclohexane-acétate d'éthyle (3—2), on sépare dans l'ordre:

—l'isomère α cristallisé. Il est trituré avec de l'ether isopropylique puis essoré et séché à l'air. On obtient 1,12 g de produit, F = 147°C. L'échantillon fond à 149°C après recristallisation dans du méthanol; $[\alpha]_D = +54° \pm 1°$ (1%, chloroforme);

— 0,65 g d'isomère β, amorphe.

EXEMPLE 5

7α-butyl 17β-hydroxy 3-oxo (17α) prègn-4-ène 21 carboxylate de potassium.

On porte au reflux durant 15 minutes 1,19 g du composé obtenu à l'exemple 4 avec un mélange de 5,4 cm3 de solution 0,53 N de potasse dans l'éthanol et de 5,4 cm3 d'eau, puis concentre sous vide jusqu'à l'obtention d'un sirop épais légèrement jaune. Par dilution avec 50 cm3 d'acétone, le sel de potassium se sépare en fines aiguilles. On l'essore, le lave à l'acétone et le sèche vers 50°C. On le recristallise par dissolution dans 3 cm3 d'acétone à 50% d'eau et addition de 28,5 cm3 d'acétone. On obtient 1,13 g d'aiguilles incolores solvatées avec deux molécules d'eau, fondant vers 260°C (Maquenne);
$[\alpha]_D = +36{,}5° \pm 1{,}5°$ (1%, eau).

EXEMPLE 6

γ-lactone de l'acide 17β-hydroxy 7α-(2-méthyl propyl) 3-oxo (17α) prègn-4-ène 21-carboxylique.

On dissout le complexe obtenu à partir de 135 mg d'iodure cuivreux et 0,25 cm3 de sulfure de n-butyle dans 15 cm3 de tétrahydrofuranne anhydre et ajoute 680 mg de canrénone (ou γ-lactone de l'acide 17β-hydroxy 3-oxo (17α) pregna 4,6-diène 21-carboxylique). La solution est refroidie à —30°C et sous bonne agitation, on ajoute, goutte à goutte, en 20 minutes, 4,3 cm3 de solution 0,7 M de bromure d'isobutylmagnésium dans l'éther éthylique. Après 15 minutes d'agitation supplémentaire à —30°C, la suspension réactionnelle se décolore du rouge orangé au jaune brun. Elle est alors acidifiée avec un large excès d'acide chlorhydrique 2N. On agite 30 minutes à la température ordinaire puis extrait avec de l'acétate d'éthyle. Les extraits sont lavés à l'eau, sèchés et évaporés à sec. Le résidu est chromatographié sur silice en utilisant un mélange benzène-éther éthylique (1—9). On sépare dans l'ordre:

— 450 mg d'isomère α qui sont recristallisés dans 1 cm3 de méthanol et donnent 320 mg de cristaux solvatés avec 0,5 mole de méthanol, fondant à 190°C. après dessolvatation vers 100°C;
$[\alpha]_D = +63° \pm 1{,}5°$ (1%, chloroforme);

— 310 mg d'isomère β. On recristallise le produit dans un mélange de chlorure de méthylène et d'éther isopropylique et obtient 230 mg de produit; F = 218°C.
$[\alpha]_D = +79° \pm 1{,}5°$ (1%, chloroforme).

EXEMPLE 7

γ-lactone de l'acide 7α-éthényl 17β-hydroxy 3-oxo (17α) prègn-4-ène 21-carboxylique.

On agite à la température ordinaire 2,1 g d'iodure cuivreux avec 5,6 cm3 de sulfure de n-butyle puis dissout le complexe formé dans une solution de 10 g de canrénone (ou de γ-lactone de l'acide 17β-hydroxy 3-oxo (17α) prégna 4,6-diène 21-carboxylique) dans 200 cm3 de tétrahydrofuranne anhydre. On refroidit la solution obtenue à —20°C et ajoute goutte à goutte en 80 minutes, sous bonne agitation, 44 cm3 de solution N de chlorure de vinylmagnésium dans le tétrahydrofuranne. On maintient sous agitation à —20°C pendant encore 60 minutes après la fin de l'addition, puis acidifie la suspension réactionnelle avec 45 cm3 d'acide chlorhydrique 2N. On agite le mélange 90 minutes à la température ordinaire, puis extrait avec de l'acétate d'éthyle. La phase organique est lavée à l'eau, avec une solution de thiosulfate de sodium 0,2 M, sèchée et distillée à sec. L'huile résiduaire est purifiée par chromatographie sur silice en utilisant un mélange cyclohexane-acétate d'éthyle (1—1).

On sépare dans l'ordre:

— 2,9 g d'isomère recherché fondant à 199°C après recristallisation dans l'acétate d'isobutyle, puis dans l'éthanol à 75°; $[\alpha]_D = +17{,}5° \pm 1{,}5°$ (1%, chloroforme);

— puis 3,5 g d'isomère β présentant un triple point de fusion vers 100, puis 142 et 161°C après recristallisation dans un mélange de chlorure de méthylène et d'éther isopropylique.

EXEMPLE 8

$\gamma$-lactone de l'acide 17$\beta$-hydroxy 3-oxo 7$\alpha$-(2-propényl) (17$\alpha$) prègn-4-ène 21-carboxylique.

a) *Préparation du complexe soluble bis (sulfure de n-butyle)-iodure cuivreux* d'après H.O. HOUSE et W.F. FISCHER, J. ORG. Chem. 33, 949, (1968).

On agite à la température ordinaire 475 mg d'iodure cuivreux avec 0,875 cm3 de sulfure de n-butyle. La dissolution du sel cuivreux, légèrement exothermique, est totale en 10 minutes. Le complexe ainsi formé (liquide jaune-orangé) est dissous dans 10 cm3 d'éther éthylique anhydre.

b) *Préparation du di-allylcuprate de lithium* selon D. SEYFERTH et M.A. WEINER, J. Org. Chem. *26*, 4797, (1961) et G.M. WHITESIDES, W.F. FISCHER Jr, J. SAN FILIPPO Jr, R.W. EASHE et H.O. HOUSE, J. AMER. Chem. soc., *91*, 4871, (1969).

A une solution de 1,95 g de triphénylallylétain dans 15 cm3 d'éther éthylique anhydre, on ajoute sous agitation 2,85 cm3 de solution 1,75 M de phényllithium dans un mélange benzène-éther éthylique 7:3. Il se forme immédiatement un précipité blanc abondant de tétraphénylétain. On agite encore 15 minutes la suspension puis la refroiduit vers −30°C, y introduit en 10 minutes la solution éthérée du complexe préparé ci-dessus et poursuit l'agitation pendant encore 15 minutes. On obtient ainsi une solution de di-allylcuprate de lithium contenant du tétraphénylétain en suspension.

c) *Réaction avec le stéroïde*

Au mélange précédent, on ajoute une solution de 680 mg de canrénone (ou $\gamma$-lactone de l'acide 17$\beta$-hydroxy 3-oxo (17$\alpha$)-pregna 4,6-diène 21-carboxylique) dans 25 cm3 de tétrahydrofuranne anhydre. Après 90 minutes d'agitation à −30°C, le mélange réactionnel est acidifié avec 15 cm3 d'acide chlorhydrique 2N, puis on poursuit l'agitation à la température ordinaire pendant 2 heures.

On sépare le tétraphénylétain par filtration, décante la phase organique, la lave à l'eau, la sèche et la distille à sec. L'extrait sec est purifié par chromatographie sur silice en utilisant le mélange chloroforme-acétate d'éthyle 95:5. On recueille 408 mg du composé recherché fondant à 184°C, puis à 194°C après resolidification. L'échantillon analytique est obtenu par recristallisation dans du méthanol. F. = 196°C.

$[\alpha]_D = +13° \pm 3°$ (0,7%, Chloroforme)

EXEMPLE 9

$\gamma$-lactone de l'acide 7$\alpha$-(3-buténýl) 17$\beta$-hydroxy 3-oxo 17$\alpha$-pregn-4-ène 21-carboxylique.

On recouvre 3 g de tournure de magnésium par 25 cm3 d'éther, puis, sous agitation, ajoute environ 10 cm3 d'une solution de 9,05 g de chlorométhyl cyclopropane dans 75 cm3 d'éther. Lorsque la réaction a démarré, on ajoute le reste de la solution ci-dessus en 40 minutes, en maintenant le reflux. On maintient ensuite sous agitation au reflux pendant 30 minutes, puis laisse reposer à température ambiante.

On prépare, par ailleurs, un complexe par dissolution de 135 mg de chlorure cuivreux et de 85 mg de chlorure de lithium dans 35 cm3 de tétrahydrofuranne. On ajoute à cette solution 1,7 g de canrénone, puis refroidit, après dissolution à −30°C.

On introduit ensuite en 30 minutes, sous agitation, 14,2 cm3 de la solution de magnésien ci-dessus, maintient ensuite l'agitation pendant 15 minutes à −30°C, puis acidifie par addition de 15 cm3 d'acide chlorhydrique 5N. On laisse revenir à température ambiante, agite le mélange jusqu'à apparition de deux phases limpides, puis extrait à l'acétate d'éthyle. On lave la phase organique à l'eau, avec une solution aqueuse saturée de chlorure de sodium, sèche et concentre à sec. On chromatographie le résidu sur silice en éluant au mélange éther-benzène (75—25). On isole ainsi:

— 1,25 g d'isomère $\alpha$ recherché que l'on peut purifier par recristallisation dans l'isopropanol ou l'acétate d'éthyle. F. = 180°C.

$[\alpha]_D = +58° \pm 1,5°$ (1%, chloroforme).

*Analyse:* $C_{26}H_{36}O_3$: 396,57

| | | |
|---|---|---|
| Calculé: | C% 78,74 | H% 9,15 |
| Trouvé : | 78,9 | 9,0 |

— 0,5 g d'isomère $\beta$ que l'on peut purifier par recristallisation dans l'isopropanol. F. = 132°C.

$[\alpha]_D = +64° \pm 1,5°$ (1%, chloroforme).

*Analyse:* $C_{26}H_{36}O_3$: 396,57

| | | |
|---|---|---|
| Calculé: | C% 78,74 | H% 9,15 |
| Trouvé : | 78,5 | 9,0 |

EXEMPLE 10

7$\alpha$-(3-buténýl) 17$\beta$-hydroxy 3-oxo 17$\alpha$-pregn-4-ène 21-carboxylate de potassium

A 2 g de l'isomère 7$\alpha$ obtenu à l'exemple 9, on ajoute 7 cm3 d'eau et 3,5 cm3 de potasse éthanolique 1,38 N. On porte au reflux pendant 30 minutes, puis évapore à sec. On ajoute de l'acétone au résidu, essore, lave à l'acétone et sèche le produit. On obtient 2,2 g de produit attendu.

$[\alpha]_D = +39° \pm 1°$ (1%, eau).

*Analyse:* $C_{26}H_{37}O_4K$ 1,25 $H_2O$

| | | |
|---|---|---|
| Calculé: | C% 65,72 | H% 8,38 |
| Trouvé : | 65,7 | 8,3 |

EXEMPLE 11

$\gamma$-lactone de l'acide 17$\beta$-hydroxy 3-oxo 7$\alpha$-phényl méthyl 17$\alpha$-pregn-4-ène 21-carboxylique

On mélange 135 mg de chlorure cuivrique, 84 mg de chlorure de lithium et 5 cm3 de tétrahydrofuranne. On y ajoute 1,7 g de canrénone dans 30 cm3 de tétrahydrofuranne, puis refroidit à 0°C environ et ajoute en 40 minutes 14,3 cm3 d'une solution 0,7 M de bromure de benzylmagnésium dans l'éther. On acidifie ensuite par addition de 10 cm3 d'acide chlorhydrique 5N, agite pendant 1 heure à température ambiante, dilue á l'eau et extrait à l'acétate d'éthyle. On lave la phase organique à l'eau, puis avec une solution aqueuse saturée de chlorure de sodium, sèche et concentre à sec. On chromatographie le résidu sur silice en éluant au mélange benzène-acétate d'éthyle (70—30) et obtient:

— 1,2 g d'isomère $\alpha$ attendu que l'on peut recristalliser dans la méthyléthylcétone. F. = 258°C. $[\alpha]_D = +2,5° \pm 1°$ (1%, chloroforme).

— 0,15 g d'isomère $\beta$ que l'on peut recristalliser dans l'éthanol. F. = 220°C.

EXEMPLE 12

Composition pharmaceutique.

On a préparé des comprimés à 50 mg de produit de exemple 2, comme principe actif.

Produit de l'exemple 2 50 mg

Excipient (talc, amidon, stéarate de magnésium).

EXEMPLE 13

Composition pharmaceutique.

On a préparé des comprimés à 50 mg de produit de l'exemple 2, comme principe actif.

Produit de l'exemple 3 30 mg

Excipient (talc, amidon, stéarate de magnésium).

*Etude pharmacologique des produits des exemples 2 et 3 et de l'homologue 7$\alpha$-méthyl du produit de l'exemple 2 (décrit dans J. Org. Chem. Soc. 26, 3077—83 (1961) dénommés ci-après produits A, B et C.*

I/ *Etude de l'activité anti-aldostérone:*

1) *Produits A et C*

L'étude a été effectuée au moyen d'un test inspiré de KAGAWA C.M. (P.S.E.B.M. 1958, *99*, 705) et de MARCUS (Endocrinology 1952, *50,* 286).

La Technique utilisée est la suivante:

Des rats mâles Sprague Dawley SPF IFFA CREDO, de 180 g, sont surrénalectomisés sept jours avant la diurèse, sous anesthésie à l'Imalgène (Kétamine) par voie intrapéritonéale à raison de 100 mg/Kg. Dès l'opération et jusqu'à la veille de l'expérience, ils reçoivent comme eau de boisson du sérum physiologique.

Les animaux sont mis à jeun dix-sept heures avant la diurèse, le sérum physiologique est alors remplacé par de l'eau glucosée à 5%.

Les produits sont administrés par voie orale une heure avant la mise en cage.

Au moment de la mise en diurèse, les animaux reçoivent une surcharge hydrosaline par voie intrapéritonéale à raison de 5 ml par rat, de sérum, physiologique à 9% et d'autre part, 1 $\mu$g/Kg de monoacétate d'aldostérone en solution alcoolique à 2,5%, par voie sous-cutanée.

Les rats sont alors placés par deux en cage à diurèse, sans nourriture, ni biosson, pendant quatre heures.

Au bout de ce temps, on effectue une miction forcée par pression sur la vessie et mesure le volume de l'urine recueillie.

Après rinçage soigneux des cages et de la verrerie, le volume des urines est amené à 50 cm3. Sur cette solution, on effectue le dosage du sodium et du potassium urinaires par photométrie de flamme à l'autoanalyseur.

Les résultats obtenus exprimés en pourcentage d'inhibition de l'activité de 1 $\mu$g/Kg de monoacétate d'aldostérone injecté par voie sous-cutanée sur le log du rapport:

$$\frac{\text{concentration en sodium}}{\text{concentration en potassium}},$$

selon la méthode de KAGAWA Endocrinology 1960, *67* p. 125—132, sont les suivants:

| | DOSE DE PRODUIT administré par voie orale | % d'inhibition |
|---|---|---|
| Produit A | 2 mg/Kg | 47 % |
| Produit C | 2 mg/Kg | 27 % |

*CONCLUSION:* à la dose de 2 mg/Kg par voie orale, le produit A est plus actif que le produit C.

2) *Produit B*

Dans les mêmes conditions du paragraphe 1, le produit B, dès la dose de 0,4 mg/kg, a donné un pourcentage d'inhibition de 58%. Le produit B présente donc une très bonne activité anti-aldostérone.

II/ *Activité androgène:*

1) *Produits A et C*

L'activité androgène des produits A et C a été étudiée par la méthode des récepteurs hormonaux décrite par JP. RAYNAUD et Coll dans "J. Ster. Biochem" 1975 — 615—622.

*La technique est la suivante:*

La prostate, prélevée sur des rats mâles castrés 24 heures auparavant, est homogénéisée dans un tampon trométhamine 10 millimoles, saccharose 0,25 M, HCl pH 7,4.

L'homogénat est centrifugé à 105 000 g pendant une heure. Le liquide surnageant ou "cytosol" est alors ajusté de façon à avoir une dilution 1/5 (poids/volume).

On incube à 0°C pendant deux heures le cytosol avec une concentration fixe de 17$\beta$-hydroxy 17$\alpha$-méthyl estra 4,9, 11-triène 3-one tritié, désigné ci-après par produit R tritié, en présence ou non de concentration croissante de ce même produit froid, designé ci-après produit R froid, de testostérone ou du produit à tester.

On détermine au bout de deux heures la radioactivité du produit tritié lié au récepteur par la technique d'absorption au charbon-dextran (1,25%—0,625%).

On trace ensuite:

— Les courbes représentant les pourcentages de produit R trité lié en fonction du log de la concentration du produit R froid, de la testostérone ou du produit à tester ajoutés.

— Et la droite $I_{50}$ parallèle à l'axe des abcisses et d'ordonnée.

$$\frac{B}{T} = \frac{B/T\ Max. + B/T\ Min.}{2}$$

B/T Max. est le pourcentage de produit R tritié lié quand aucun produit n'est ajouté.

B/T Min. est le pourcentage de produit R tritié lié quand la quantité maximale de produit R froid est ajoutée.

Les intersections de cette droite $I_{50}$ et des courbes permettent de déterminer les valeurs CT et CX.

CT: concentration de la testostérone froide qui inhibe de 50% la fixation du produit R tritié.

CX: concentration du produit testé qui inhibe de 50% la fixation du produit R tritié.

L'affinité relative du produit testé ou ARL est donnée par la formule:

$$ARL = 100 \times \frac{CT}{CX}$$

*Les résultats sont les suivants:*

| PRODUITS | AR L |
|---|---|
| Testostérone | 100 |
| Produit A | 0,4 |
| Produit C | 28 |

*CONCLUSION:* Le produit A est pratiquement dénué d'affinité pour le récepteur prostatique de la testostérone, alors que le produit C présente une assez nette affinité pour le récepteur prostatique de la testostérone.

2) *Produit B*

Dans les mêmes conditions du paragraphe 1, le produit B a donné une ARL de 0,2. Le produit B montre donc une affinité encore inférieure à celle du produit A pour le récepteur prostatique de la testostérone.

**Revendications pour les Etats contractants: BE, CH, DE, FR, GB, IT, LU, NL et SE**

1. Les composés répondant à la formule I:

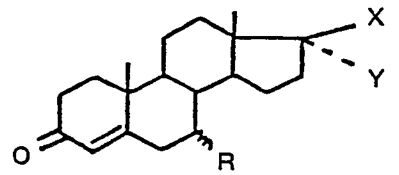

(I)

dans laquelle R, en position $\alpha$ ou en position $\beta$, représente un radical alkyle saturé ou insaturé renfermant de 2 à 8 atomes de carbone, un radical cycloalkylalkyle renfermant de 4 à 8 atomes de carbone ou un radical arylalkyle renfermant de 7 à 12 atomes de carbone et,

— ou bien X et Y représentent le groupement:

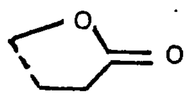

— ou bien X représente le groupement OH et Y le groupement

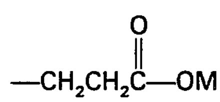

dans lequel M représente un atome d'hydrogène, ou un atome de métal alcalin ou un groupement $NH_4$.

2. Les composés de formule I, tels que définis à la revendication 1, pour lesquels X et Y représentent le groupement:

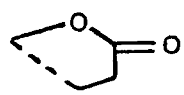

3. Les composés de formule I, tels que définis à la revendication 1, pour lesquels X représente un radical hydroxyle et Y un radical

$$-CH_2CH_2CO_2K.$$

4. Les composés de formule I, tels que définis à la revendication 1, 2 ou 3, pour lesquels le radical R est en position $7\alpha$.

5. Les composés de formule I tels que définis à l'une quelconque des revendications 1 à 4 pour lesquels R représente un radical éthyle, n-propyle, n-butyle ou 2-méthyl-propyle.

6. Les composés de formule I, tels que définis à l'une quelconque des revendications 1 à 4, pour lesquels R représente le radical vinyle ou allyle.

7. La $\gamma$-lactone de l'acide $17\beta$-hydroxy 3-oxo $7\alpha$-propyl $(17\alpha)$ pregn-4-ène 21-carboxylique et le $17\beta$-hydroxy 3-oxo $7\alpha$-propyl $(17\alpha)$ pregn-4-ène 21-carboxylate de potassium.

8. A titre de médicament, les composés de formule I, tels que définis à l'une quelconque des revendications 1 à 6.

9. A titre de médicaments, la $\gamma$-lactone de l'acide $17\beta$-hydroxy 3-oxo $7\alpha$-propyl $(17\alpha)$ pregn-4-ène 21-carboxylique et le $17\beta$-hydroxy 3-oxo $7\alpha$-propyl $(17\alpha)$ pregn-4-ène 21-carboxylate de potassium.

10. Les compositions pharmaceutiques renfermant comme principe actif au moins un médicament défini à l'une quelconque des revendications 8 et 9.

11. Procédé de préparation des composés de formule I, tels que définis à l'une quelconque des revendications 1 à 6, caractérisé en ce que l'on soumet le composé de formule II:

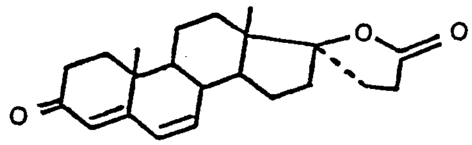

(II)

d'abord à l'action d'un composé de formule III:

RMg Hal (III)

dans laquelle R conserve la même signification que dans la revendication 1 et Hal représente un atome d'halogène, en présence d'un sel cuivreux, ou d'un composé de formule IV:

$(R)_2$ Cu Li (IV)

dans laquelle R conserve la même signification que dans la revendication 1, puis à l'action d'un acide, pour obtenir un composé de formule $I_A$:

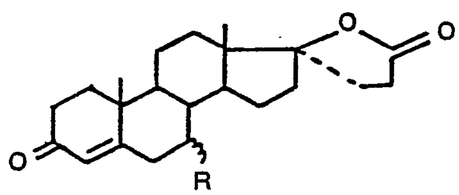

$I_A$

éventuellement sous forme d'un mélange d'isomères $7\alpha$ et $7\beta$ que l'on sépare, si désiré, puis, si désiré, soumet soit chacun des isomères, soit leur mélange à l'action d'un hydroxyde alcalin ou de l'ammoniaque, pour obtenir un composé de formule $I_B$:

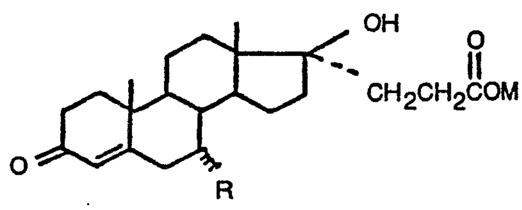

$I_B$

dans laquelle R conserve la même signification que précédemment et M représente un atome de métal alcalin ou un groupement $NH_4$, sous forme d'isomère $7\alpha$ ou $7\beta$ ou de leur mélange, que l'on sépare, si désiré, en chacun des isomères, puis soumet soit chacun des isomères ainsi obtenus, soit leur mélange à l'action d'un agent acide pour obtenir le composé correspondant de formule $I_B$ dans laquelle M représente un atome d'hydrogène.

**Revendications pour l'Etat contractant: AT**

1. Procédé de préparation des composés répondant à la formule I:

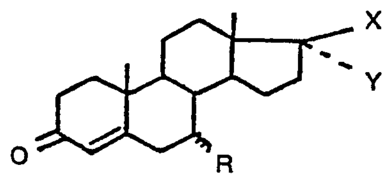

(I)

dans laquelle R, en position $\alpha$ ou en position $\beta$, représente un radical alkyle saturé ou insaturé renfermant de 2 à 8 atomes de carbone, un radical cycloalkylalkyle renfermant de 4 à 8 atomes de carbone ou un radical arylalkyle renfermant de 7 à 12 atomes de carbone et,

— ou bien X et Y représentent le groupement:

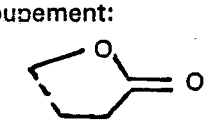

— ou bien X représente le groupement OH et Y le groupement

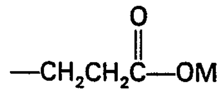

dans lequel M représente un atome d'hydrogène ou un atome de métal alcalin ou un groupement $NH_4$, caractérisé en ce que l'on soumet le composé de formule II:

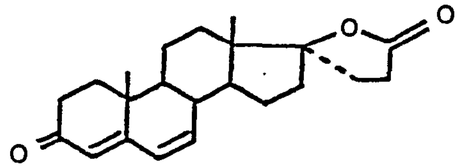

(II)

d'abord à l'action d'un composé de formule III:

RMg Hal (III)

dans laquelle R conserve la même signification que précédemment et Hal représente un atome d'halogène, en présence d'un sel cuivreux, ou d'un composé de formule IV:

$(R)_2$ Cu Li (IV)

dans laquelle R conserve la même signification que précédemment, puis à l'action d'un acide, pour obtenir un composé de formule $I_A$:

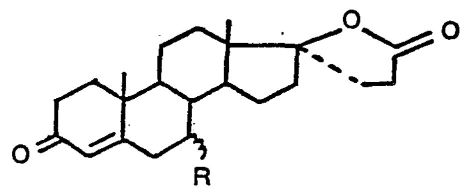

$I_A$

éventuellement sous forme d'un mélange d'isomères 7$\alpha$ et 7$\beta$ que l'on sépare, si désiré, puis, si désiré, soumet soit chacun des isomères, soit leur mélange à l'action d'un hydroxyde alcalin ou de l'ammoniaque, pour obtenir un composé de formule $I_B$:

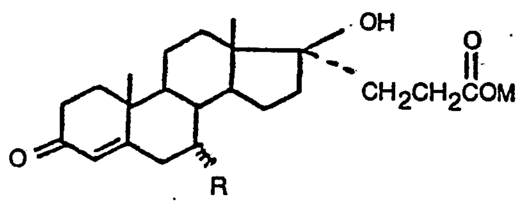

$I_B$

dans laquelle R conserve la même signification que précédemment et M représente un atome de métal alcalin ou un groupement $NH_4$, sous forme d'isomère 7$\alpha$ ou 7$\beta$ ou de leur mélange, que l'on sépare, si désiré, en chacun des isomères, puis soumet soit chacun des isomères ainsi obtenus, soit leur mélange à l'action d'un agent acide pour obtenir le composé correspondant de formule $I_B$ dans laquelle M représente un atome d'hydrogène.

2. Procédé selon la revendication 1, caractérisé en ce que, dans la formule III ou IV, R représente un radical éthyle, n-propyle, n-butyle ou 2-méthyl propyle.

3. Procédé selon la revendication 1, caractérisé en ce que, dans la formule III ou IV, R représente le radical vinyle ou allyle.

4. Procédé selon la revendication 1 ou 2, caractérisé en ce que l'on prépare la $\gamma$-lactone de l'acide 17$\beta$-hydroxy 3-oxo 7$\alpha$-propyl (17$\alpha$) pregn-4-ène 21-carboxylique et le 17$\beta$-hydroxy 3-oxo 7$\alpha$-propyl (17$\alpha$) pregn-4-ène 21-carboxylate de potassium.

**Patentansprüche für die Ventragsstaaten: BE, CH, DE, FR, GB, IT, CU, NL, DE**

1. Verbindungen der Formel I

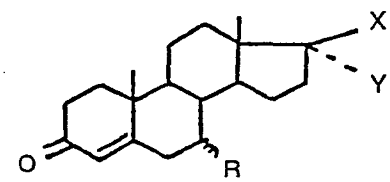

(I)

worin R in $\alpha$-Stellung oder $\beta$-Stellung einen gesättigten oder ungesättigten Alkylrest mit 2 bis 8 Kohlenstoffatomen, einen Cycloalkylalkylrest mit 4 bis 8 Kohlenstoffatomen oder einen Aralkylrest mit 7 bis 12 Kohlenstoffatomen bedeutet und entweder X und Y die Gruppe

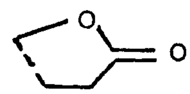

bedeuten
oder X die Gruppe OH bedeutet und Y die Gruppe

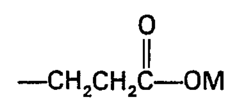

bedeutet, worin M ein Wasserstoffatom oder ein Alkalimetallatom oder eine Gruppe $NH_4$ darstellt.

2. Verbindungen der Formel I gemäß Anspruch 1, worin X und Y die Gruppe

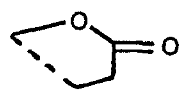

darstellen.

3. Verbindungen der Formel I gemäß Anspruch 1, worin X einen Hydroxylrest bedeutet und Y einen Rest

$$-CH_2CH_2CO_2K$$

darstellt.

4. Verbindungen der Formel I gemäß Anspruch 1, 2 oder 3, worin der Rest R in $7\alpha$-Stellung vorliegt.

5. Verbindungen der Formel I gemäß einem der Ansprüche 1 bis 4, worin R einen Äthyl-, n-Propyln-Butyl- oder 2-Methyl-propylrest bedeutet.

6. Verbindungen der Formel I gemäß einem der Ansprüche 1 bis 4, worin R den Vinyl- oder Allylrest darstellt.

7. $\gamma$-Lacton der $17\beta$-Hydroxy-3-oxo-$7\alpha$-propyl-($17\alpha$)pregn-4-en-21-carbonsäure und das Kalium-$17\beta$-hydroxy-3-oxo-$7\alpha$-propyl-($17\alpha$)-pregn-4-en-21-carboxylat.

8. Als Arzneimittel die Verbindungen der Formel I gemäß einem der Ansprüche 1 bis 6.

9. Als Arzneimittel das $\gamma$-Lacton der $17\beta$-Hydroxy-3-oxo-$7\alpha$-propyl-($17\alpha$)-pregn-4-en-21-carbonsäure und das Kalium-$17\beta$-hydroxy-3-oxo-$7\alpha$-propyl-($17\alpha$)-pregn-4-en-21-carboxylat.

10. Pharmazeutische Zusammensetzungen enthaltend als Wirkstoff zumindest ein Arzneimittel gemäß einem der Ansprüche 8 und 9.

11. Verfahren zur Herstellung der Verbindungen der Formel I gemäß einem der Ansprüche 1 bis 6, dadurch gekennzeichnet, daß man die Verbindung der Formel II

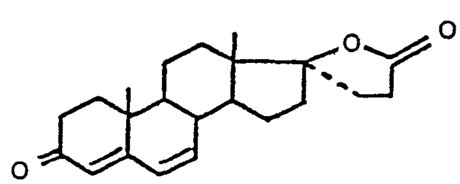

(II)

zunächst der Einwirkung einer Verbindung der Formel III

RMgHal (III)

worin R die in Anspruch 1 angegebene Bedeutung besitzt und Hal ein Halogenatom bedeutet, in Gegenwart eines Kupfersalzes bzw. Kupfer(I)-salzes oder einer Verbindung der Formel IV

$$(R)_2CuLi \qquad (IV)$$

worin R die in Anspruch 1 angegebene Bedeutung besitzt und danach der Einwirkung einer Säure unterzieht, um eine Verbindung der Formel $I_A$

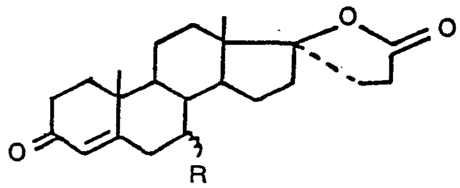

$I_A$

gegebenenfalls in Form eines Gemisches der 7$\alpha$ und 7$\beta$-Isomeren, das man gewünschtenfalls trennt, zu erhalten, danach gewünschtenfalls entweder ein jedes der Isomeren oder ihr Gemisch der Einwirkung eines Alkalihydroxyds oder der Einwirkung von Ammoniak unterzieht, um eine Verbindung der Formel $I_B$

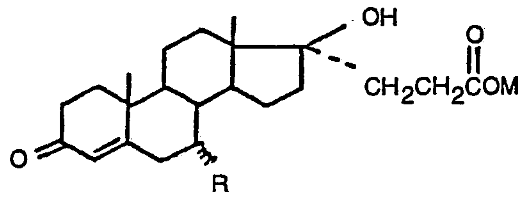

$I_B$

worin R die vorstehend angegebene Bedeutung besitzt und M ein Alkalimetallatom oder eine Gruppe $NH_4$ bedeutet, in Form des 7$\alpha$- oder 7$\beta$-Isomeren oder in Form von deren Gemisch zu erhalten, das man gewünschtenfalls in ein jedes der Isomeren auftrennt und danach entweder ein jedes der so erhaltenen Isomeren oder ihr Gemisch der Einwirkung eines sauren Mittels unterzieht, um die entsprechende Verbindung der Formel $I_B$ zu erhalten, worin M ein Wasserstoffatom bedeutet.

**Patentansprüche für den Ventragsstaat: AT**

1. Verfahren zur Herstellung der Verbindung der Formel I

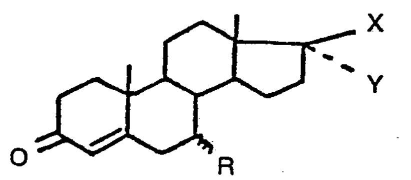

(I)

worin R in $\alpha$- oder $\beta$-Stellung einen gesättigten oder ungesättigten Alkylrest mit 2 bis 8 Kohlenstoffatomen, einen Cycloalkylalkylrest mit 4 bis 8 Kohlenstoffatomen oder einen Aralkylrest mit 7 bis 12 Kohlenstoffatomen bedeutet und
entweder X und Y die Gruppe

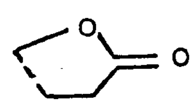

bedeuten
oder X die Gruppe OH bedeutet und Y die Gruppe

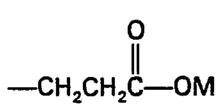

bedeutet, worin M ein Wasserstoffatom oder ein Alkalimetallatom oder eine Gruppe $NH_4$ darstellt, dadurch gekennzeichnet, daß man die Verbindung der Formel II

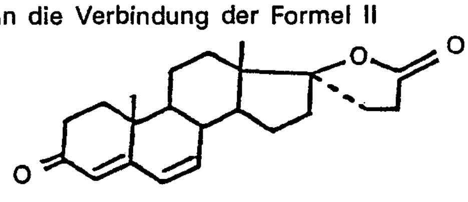

(II)

zunächst der Einwirkung einer Verbindung der Formel III

RMgHal (III)

worin R die vorstehend angegebene Bedeutung besitzt und Hal ein Halogenatom bedeutet, in Gegenwart eines Kupfersalzes bzw. Kupfer(I)-salzes oder einer Verbindung der Formel IV

$(R)_2CuLi$ (IV)

worin R die vorstehend angegebene Bedeutung besitzt und danach der Einwirkung einer Säure unterzieht, um eine Verbindung der Formel $I_A$

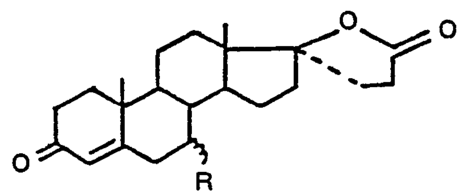

$I_A$

gegebenenfalls in Form eines Gemisches der 7$\alpha$- und 7$\beta$-Isomeren, das man gewünschtenfalls auf trennt, zu erhalten und danach gewünschtenfalls entweder ein jedes der Isomeren oder Ihr Gemisch der Einwirkung eines Alkalihydroxyds oder der Einwirkung von Ammoniak unterzieht, um eine Verbindung der Formel $I_B$

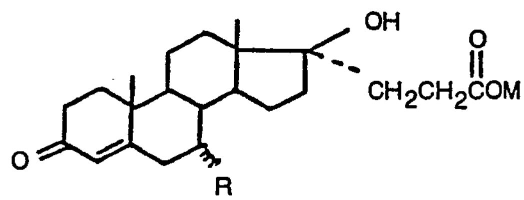

$I_B$

worin R die vorstehend angegebene Bedeutung besitzt und M ein Alkalimetallatom oder eine Gruppe $NH_4$ bedeutet, in Form des 7$\alpha$- oder 7$\beta$-Isomeren oder in Form von deren Gemisch, das man gewünschtenfalls in ein jedes der Isomeren auftrennt, zu erhalten und danach entweder ein jedes der so erhaltenen Isomeren oder ihr Gemisch der Einwirkung eines sauren Mittels untezieht, um die entsprechende Verbindung der Formel $I_B$ zu erhalten, worin M ein Wasserstoffatom bedeutet.

2. Verfahren gemäß Anspruch 1, dadurch gekennzeichnet, daß in der Formel III oder IV R einen Äthyl-, n-Propyl-, n-Butyl- oder 2-Methylpropylrest bedeutet.

3. Verfahren gemäß Anspruch 1, dadurch gekennzeichnet, daß in der Formel III oder IV R den Vinyl- oder Allylrest bedeutet.

4. Verfahren gemäß Anspruch 1 oder 2, dadurch gekennzeichnet, daß man das $\gamma$-Lacton der 17$\beta$-Hydroxy-3-oxo-7$\alpha$-propyl-(17$\alpha$)-pregn-4-en-21-carbonsäure und das Kalium-17$\beta$-hydroxy-3-oxo-7$\alpha$-propyl-(17$\alpha$)-pregn-4-en-21-carboxylat herstellt.

**Claims for the Contracting States: BE, CH, DE, FR, GB, IT, LU, NL, SE.**

1. The compounds corresponding to the formula I:

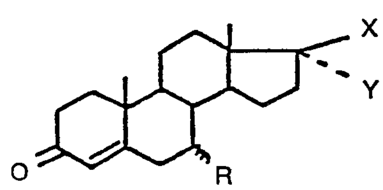

(I)

in which R, at position $\alpha$ or at position $\beta$, represents a saturated or unsaturated alkyl radical containing from 2 to 8 carbon atoms, a cycloalkylalkyl radical containing from 4 to 8 carbon atoms or an arylalkyl radical containing from 7 to 12 carbon atoms and

— either X and Y represent the group:

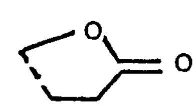

— or X represents the group OH and Y represents the group:

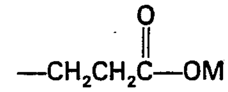

in which M represents a hydrogen atom or an alkali metal atom or a group $NH_4$.

2. The compounds of formula I, as defined in Claim 1, for which X and Y represent the group:

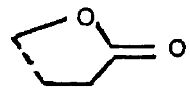

3. The compounds of formula I, as defined in Claim 1, for which X represents a hydroxyl radical and Y represents a radical $—CH_2CH_2CO_2K$.

4. The compounds of formula I, as defined in Claim 1, 2 or 3, for which the radical R is at position 7$\alpha$.

5. The compounds of formula I, as defined in any one of Claims 1 to 4, for which R represents an ethyl, n-propyl, n-butyl or 2-methyl-propyl radical.

6. The compounds of formula I, as defined in any one of Claims 1 to 4, for which R represents the vinyl or allyl radical.

7. 17$\beta$-hydroxy 3-oxo 7$\alpha$-propyl (17$\alpha$) pregn-4-ene 21-carboxylic acid $\gamma$-lactone and potassium 17$\beta$-hydroxy 3-oxo 7$\alpha$-propyl (17$\alpha$) pregn-4-ene 21-carboxylate.

8. As medicaments the compounds of formula I, as defined in any one of Claims 1 to 6.

9. As medicaments 17$\beta$-hydroxy 3-oxo 7$\alpha$-propyl (17$\alpha$) pregn-4-ene 21-carboxylic acid $\gamma$-lactone and potassium 17$\beta$-hydroxy 3-oxo 7$\alpha$-propyl (17$\alpha$) pregn-4-ene 21-carboxylate.

10. The pharmaceutical compositions containing, as active principle, at least one medicament defined in any one of Claims 8 and 9.

11. Process for preparing the compounds of formula I, as defined in any one of Claims 1 to 6, characterized in that the compound of formula II:

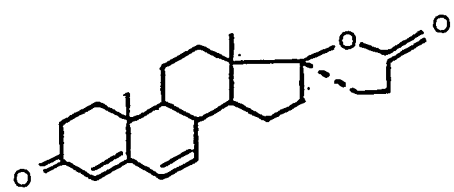

(II)

is subjected firstly to the action of a compound of formula III:

RMg Hal (III)

in which R keeps the same meaning as in Claim 1 and Hal represents a halogen atom, in the presence of a cuprous salt, or of a compound of formula IV:

$(R)_2$ Cu Li (IV)

in which R keeps the same meaning as in Claim 1, then to the action of an acid, to obtain a compound of formula $I_A$:

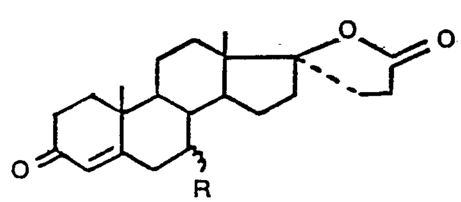

($I_A$)

possibly in the form of a mixture of isomers 7$\alpha$ and 7$\beta$ which are separated, if desired, then, if desired, either each of the isomers or the mixture thereof is subjected to the action of an alkaline hydroxide or of ammonia, to obtain a compound of formula $I_B$:

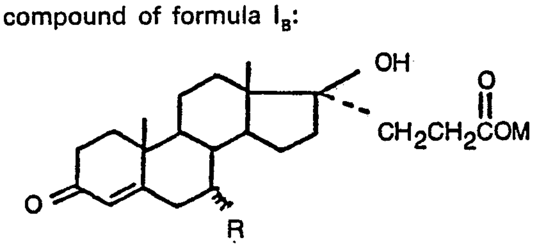

($I_B$)

in which R keeps the same meaning as before and M represents an alkali metal atom or a group $NH_4$, in the form of isomer 7$\alpha$ or 7$\beta$ or the mixture thereof, which is separated, if desired, into each of the isomers, then either each of the isomers thus obtained, or the mixture thereof, is subjected to the action of an acid agent to obtain the corresponding compound of formula $I_B$ in which M represents a hydrogen atom.

**Claims for the Contracting State: AT.**

1. Process for preparing the compounds corresponding to the formula:

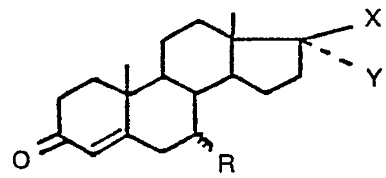

(I)

in which R, at position $\alpha$ or at position $\beta$, represents a saturated or unsaturated alkyl radical containing from 2 to 8 carbon atoms, a cycloalkylalkyl radical containing from 4 to 8 carbon atoms or an arylalkyl radical containing from 7 to 12 carbon atoms and

— either X and Y represent the group:

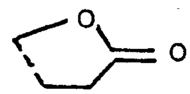

— or X represents the group OH and Y represents the group:

$$-CH_2CH_2\overset{\overset{O}{\|}}{C}-OM$$

in which M represents a hydrogen atom or an alkali metal atom or a group $NH_4$, characterized in that the compound of formula II:

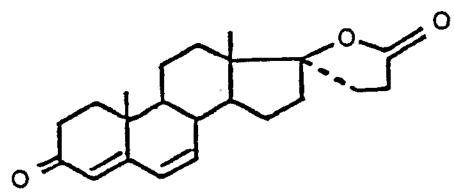

(II)

is subjected firstly to the action of a compound of formula III:

RMg Hal (III)

in which R keeps the same meaning as before and Hal represents a halogen atom, in the presence of a cuprous salt, or of a compound of formula IV:

$(R)_2$ Cu Li (IV)

in which R keeps the same meaning as before, then to the action of an acid, to obtain a compound of formula $I_A$:

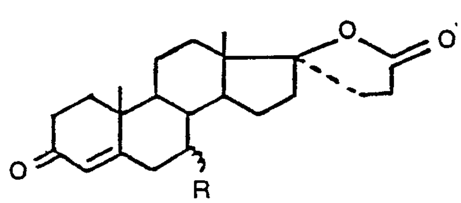

($I_A$)

possibly in the form of a mixture of isomers 7$\alpha$ and 7$\beta$, which are separated, if desired, then, if desired, either each of the isomers or the mixture thereof is subjected to the action of an alkaline hydroxide or of ammonia, to obtain a compound of formula $I_B$:

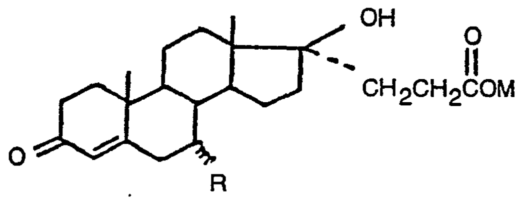

(I$_B$)

in which R keeps the same meaning as before and M represents an alkali metal atom or a group $NH_4$, in the form of isomer 7$\alpha$ or 7$\beta$ or of the mixture thereof, which is separated, if desired, into each of the isomers, then either each of the isomers thus obtained or the mixture thereof is subjected to the action of an acid agent to obtain the corresponding compound of formula $I_B$, in which M represents a hydrogen atom.

2. Process according to Claim 1, characterized in that, in the formula III or IV, R represents an ethyl, n-propyl, n-butyl or 2-methyl-propyl radical.

3. Process according to Claim 1, characterized in that, in the formula III or IV, R represents the vinyl or allyl radical.

4. Process according to Claim 1 or 2, characterized in that 17$\beta$-hydroxy 3-oxo 7$\alpha$-propyl (17$\alpha$) pregn-4-ene 21-carboxylic acid $\gamma$-lactone and potassium 17$\beta$-hydroxy 3-oxo 7$\alpha$-propyl (17$\alpha$) pregn-4-ene 21-carboxylate are prepared.